# EUROPÄISCHE PATENTANMELDUNG

(11) **EP 1 338 609 A1**
(43) Veröffentlichungstag der Anmeldung: **27.08.2003**
(21) Anmeldenummer: 03003865.7
(22) Anmeldetag: 20.02.2003
(51) Int. Cl.: C07K 14/705, C07K 16/28, A01K 67/027, A61K 38/17, A61K 39/395, A61K 31/70, C12N 5/06, G01N 33/50, C12N 5/10

(54) **Ein weiterer KDR Rezeptor und seine Anwendung**

(30) Priorität: 21.02.2002 EP 02003901
(71) Anmelder: MEMOREC Stoffel GmbH-Medizinisch-Molekulare Entwicklung, Köln, 50829 Köln (DE)
(72) Erfinder: Hofmann, Kay, 50823 Köln (DE); Peters, Bettina, 50668 Köln (DE)
(74) Vertreter: Schreiber, Christoph, Dr.

(57) **Zusammenfassung**

Die Erfindung betrifft den KDR Rezeptor und seine Anwendung.

## Beschreibung

Die vorliegende Erfindung betrifft Nukleinsäuren, die für den KDR Rezeptor kodieren, und ihre Anwendung.

Ein biologisch und medizinisch wichtiger Mechanismus des programmierten Zelltodes ist die Rezeptor-vermittelte Apoptose. Eine kurze Beschreibung der grundlegenden Prinzipien folgt, eine detailliertere Beschreibung findet sich in einer Reihe von Übersichtsartikeln (Nagata 1997; Schulze-Osthoff et al. 1998). Die Rezeptor-vermittelte Apoptose dient dazu, nicht mehr benötigte Zellen, oder solche, die dem Organismus Schaden zufügen können, in einer geordneten Art und Weise zu entfernen. Hierbei ist es zu vermeiden, dass potentiell gefährliche Inhaltsstoffe der Zelle durch Platzen der einhüllenden Membran freigesetzt werden.

In einem ersten Schritt wird die Zelle, deren Apoptose wünschenswert ist, durch einen speziellen Liganden zur Selbst-Vernichtung angeregt. Eine Reihe solcher Liganden sind bekannt, die alle zu einer bestimmten Protein-Familie gehören. Beispiele sind der Tumor-Nekrose Faktor (TNF), der Fas-Ligand (FasL) und der TNF-verwandte Ligand TRAIL. Apoptose-induzierende Liganden können sich entweder an der Oberfläche der signalgebenden Zelle befinden, oder sie können lösliche Protein sein, die von der signalgebenden Zelle sezerniert werden. Typische Liganden der TNF-Familie bilden homotypische Komplexe, i.d.R. liegen sie als Trimere vor.

Die Apoptose-induzierenden Liganden binden in der Folge an bestimmte Rezeptoren an der Oberfläche der Zielzelle. Der extrazelluläre Anteil der Rezeptoren, der den Liganden bindet, formt dabei ebenfalls einen trimeren Komplex. Die durch die Liganden-Bindung hervorgerufene Konformationsänderung des Rezeptors führt dazu, dass der intrazelluläre Anteil das Signal in das Innere der Zielzelle weitergibt.

Eine Reihe von Apoptose-induzierenden Rezeptoren ist bereits bekannt. Bekannte Beispiele für Apoptose-induzierenden Rezeptoren sind Fas, TNF-R1, TRAMP (DR3), TRAIL-R1 (DR4) und TRAIL-R2 (DR5). Eine komplette Übersicht über die Rezeptoren und die zugehörigen Liganden findet sich in (Bodmer et al. 2002; Locksley et al. 2001). Alle bekannten Apoptose-induzierenden Rezeptoren gehören einer einzigen Protein-Familie an, der TNF-Rezeptor Familie. Ein kennzeichnendes Merkmal der Mitglieder dieser Familie ist das Vorliegen spezieller Cystein-reicher Domänen (CRDs) in extrazellulären Anteil der Rezeptors. Typische CRDs enthalten sechs Cystein-Reste einem Bereich von 35-60 Aminosäuren, die die Struktur der CRDs durch die Bildung von drei Disulfid-Brücken stabilisieren (Bodmer et al. 2002). Es gibt jedoch auch atypische Varianten mit weniger als sechs Cystein-Resten. Die CRDs dieser Rezeptor-Familie dienen dazu, den entsprechenden Liganden mit hoher Spezifität zu binden und die damit verbundene Konformations-Änderung einzuleiten.

Während die TNF-Rezeptor Familie mehr als 25 Mitglieder umfasst, ist nur eine kleine Subfamilie in der Lage, Apoptose zu induzieren. Andere Mitglieder der Rezeptor-Familie signalisieren andere zelluläre Ereignisse. Ein Kennzeichen der Apoptose-induzierenden Subgruppe ist das Vorliegen einer sogenannten 'death domain' im intrazellulären Anteil des Rezeptors. Die 'death domain' ist ein schwach koriserviertes Motiv von etwa 100 Aminosäuren, das die Funktion hat bestimmte intrazelluläre Adaptor-Proteine zu binden und so das Apoptose-Signal weiterzuleiten (Hofmann and Tschopp 1995). Obwohl alle Apoptose-induzierenden Mitglieder der TNF-Rezeptor Familie zwei konservierte Motive aufweisen (die CRDs und die death domain), ist die Identifizierung neuer Rezeptoren dieser Familie schwierig. Beide Motive sind zu schwach konserviert, um eine direkte Ähnlichkeit bei üblichen Verfahren der Sequenzanalyse erkennen zu lassen. Eine Reihe von Apoptose-induzierenden Rezeptoren ist auch in der Lage, andere zelluläre Ereignisse zu signalisieren, oftmals ist das genaue Signal abhängig vom Zelltyp und von anderen biologischen Parametern.

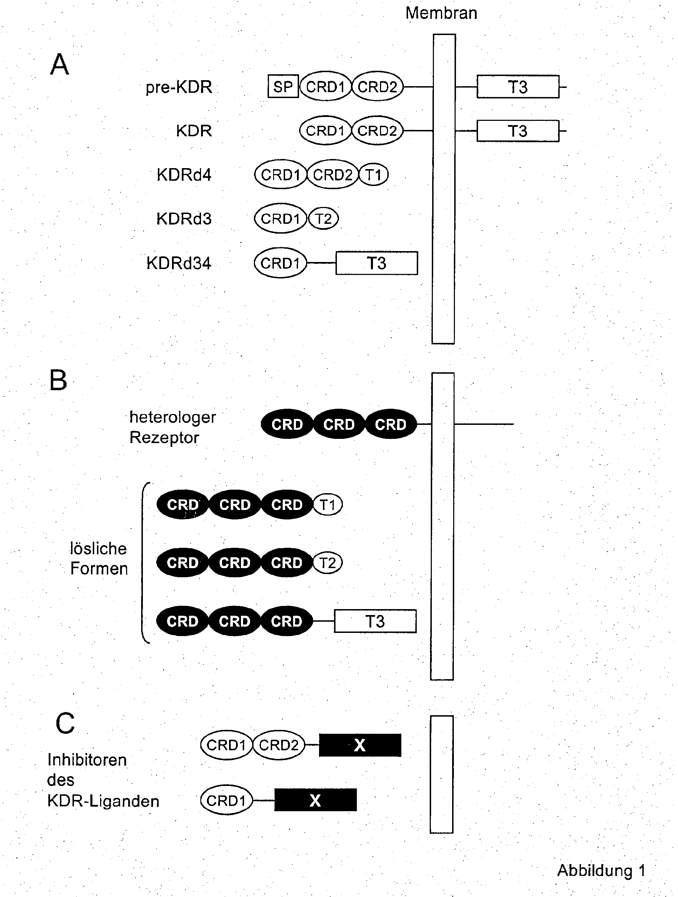

Durch die vorliegende Erfindung werden erstmals Nukleinsäuren, codierend für KDR, einen neuartigen Apoptose-induzierenden Rezeptor des Vertebraten verfügbar gemacht. Der KDR Rezeptor ist dadurch charakterisiert, dass es ein Typ I Transmembranprotein ist, im extrazellulären Bereich zwei atypische CRD-Bereiche enthält, und im intrazellulären Bereich eine ebenfalls atypische 'death domain' enthält. Neben dieser kompletten Form des KDR Rezeptors werden auch eine Reihe von splice-Varianten offenbart, die eine geänderte mRNA-Sequenz und damit auch eine geänderte Aminosäure-Sequenz enthalten. Die Architektur des KDR-Rezeptors und seiner splice-Varianten ist in Figur 1A gezeigt.

Figur 1A zeigt die Architektur der verschiedenen biologisch aktiven Formen des KDR Rezeptor Proteins. Da die Architektur des humanen und murinen KDR Rezeptors analog sind, gelten die Darstellungen für beide Spezies. Das oberste Modell repräsentiert den kompletten KDR Rezeptor. Er enthält ein N-terminales Signalpeptid, zwei Cystein-reiche Domänen (CRDs), eine membrandurchspannende Domäne, sowie eine C-terminal trimerisierungsdomäne, die mit T3 abgekürzt ist und zur Familie der 'death domains' (Hofmann and Tschopp 1995). Die Korrespondierenden Sequenzen sind im Sequenzreport unter den Seq. ID 1,2,9,10 gegeben.

Das zweite Modell entspricht dem biologisch aktiven Rezeptor, der durch Abspaltung des N-terminalen Signalpeptides entsteht. Die Peptidsequenz des prozessierten humanen KDR Rezeptors kann aus der in Seq. ID 1 gegebenen kompletten Sequenz durch Entfernen der 18 bis 26 N-terminalen Aminosäuren, bevorzugt der 22 N-terminalen Aminosäuren erhalten werden. Die Peptidsequenz des prozessierten murinen KDR Rezeptors kann aus der in Seq. ID 9 gegebenen kompletten Sequenz durch Entfernen der 18 bis 26 N-terminalen Aminosäuren, bevorzugt der 20 oder 22 N-terminalen Aminosäuren erhalten werden.

Das dritte, vierte und fünfte Modell entspricht drei unterschiedlichen verkürzten Formen des KDR Rezeptors. Diese Formen sind in der Lage, den kompletten KDR-Rezeptor zu antagonisieren, indem sie um die Liganden des KDR Rezeptors kompetieren. Die verkürzten Formen entstehen durch alternatives Splicing aus dem selben Genlocus wie der komplette KDR Rezeptor. Durch diesen Prozess werden die Trimerisierungsdomänen T1, T2 bzw. T3 an die Liganden-bindenden CRD-Bereiche des KDR Rezeptors angehängt. Die Peptid- und cDNA Sequenzen der verkürzten Formen des KDR-Rezeptors sind als Seq. ID 3-8 (human) und Seq. ID 11-16 (murin) gezeigt. Die biologisch aktiven Formen der verkürzten KDR Rezeptoren entstehen, wie oben für den kompletten KDR beschrieben, durch proteolytische Entfernung des N-terminalen Signalpeptides. Durch Entfernen der 18-26 N-terminalen Aminosäuren, bevorzugt aber der 22 N-terminalen Aminosäuren, können aus den in Seq. ID 3,5,7 gezeigten humanen Sequenzen die entsprechenden Peptidsequenzen der biologisch aktiven verkürzten KDR-Formen erhalten werden. Analog können durch Entfernen der 18-26 N-terminalen Aminosäuren, bevorzugt aber der 20 oder 22 N-terminalen Aminosäuren, aus den in Seq. ID 11,13,15 gezeigten murinenen Sequenzen die entsprechenden Peptidsequenzen der biologisch aktiven verkürzten KDR-Formen erhalten werden.

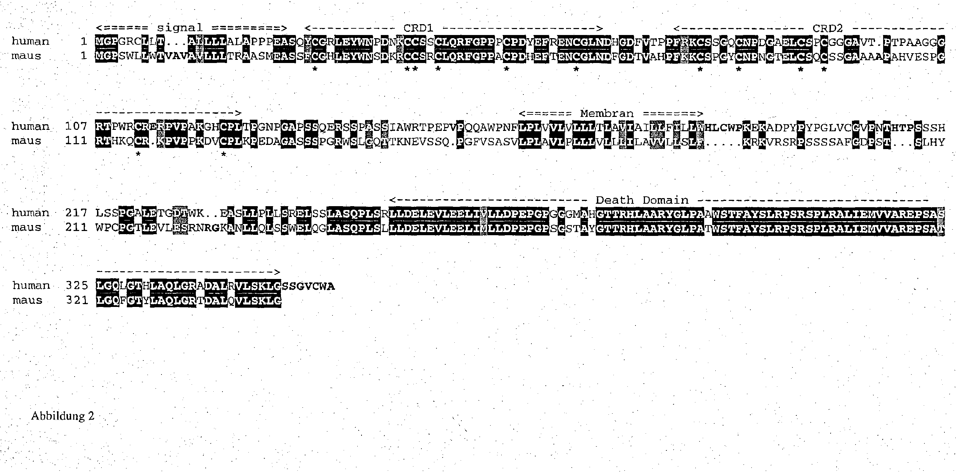

Die Position der funktionalen Domänen, wie z.B. das bevorzugte Signalpeptid, die Cystein-reichen Domänen (CRDs), die membrandurchspannende Domäne und die 'death domain' sind in Figur 2 am Beispiel eines Alignments der kompletten humanen und murinen KDR Protein-Sequenzen gezeigt.

Erklärung des Sequenz-Reports:
**Seq. ID 1** ist die komplette Peptid-Sequenz des humanen KDR Rezeptors.
**Seq. ID 2** ist die cDNA Sequenz des kompletten humanen KDR Rezeptors.
**Seq. ID 3** ist die Peptid-Sequenz eines verkürzten humanen KDR Rezeptors (d3 Form).
**Seq. ID 4** ist die cDNA Sequenz eines verkürzten humanen KDR Rezeptors (d3 Form)
**Seq. ID 5** ist die Peptid-Sequenz eines verkürzten humanen KDR Rezeptors (d4 Form).
Seq. ID 6 ist die cDNA Sequenz eines verkürzten humanen KDR Rezeptors (d4 Form)
Seq. ID 7 ist die Peptid-Sequenz eines verkürzten humanen KDR Rezeptors (d34 Form).
**Seq. ID 8** ist die cDNA Sequenz eines verkürzten humanen KDR Rezeptors (d34 Form)
**Seq. ID 9** ist die komplette Peptid-Sequenz des murinen KDR Rezeptors.
**Seq. ID 10** ist die cDNA Sequenz des kompletten murinen KDR Rezeptors.
Seq. ID 11 ist die Peptid-Sequenz eines verkürzten murinen KDR Rezeptors (d3 Form).
Seq. ID 12 ist die cDNA Sequenz eines verkürzten murinen KDR Rezeptors (d3 Form)
Seq. ID 13 ist die Peptid-Sequenz eines verkürzten-murinen KDR Rezeptors (d4 Form).
Seq. ID 14 ist die cDNA Sequenz eines verkürzten murinen KDR Rezeptors (d4 Form)
Seq. ID 15 ist die Peptid-Sequenz eines verkürzten murinen KDR Rezeptors (d34 Form).
Seq. ID 16 ist die cDNA Sequenz eines verkürzten murinen KDR Rezeptors (d34 Form)

Bei Kenntnis der Aminosäure- und Nukleinsäurestruktur des humanen und murinen KDR Rezeptors kann der Fachmann unter Berücksichtigung der hohen Ähnlichkeit zwischen der humanen und murinen Sequenz die entsprechenden Nukleinsäuren und Proteine aus anderen Vertebraten, insbesondere aus anderen Säugetieren, leicht auffinden. Dazu kann er zum einen kreuzreagierende Antikörper für eine spezifische affinitätschromatographische Aufreinigung einsetzen, oder er kann auf der Grundlage der Nukleinsäuresequenz Oligonukleotidprimer synthetisieren und die gesuchten Nukleinsäuren mit Hilfe der Polymerasekettenreaktion in einer cDNA-Bank des Eukaryonten amplifizieren. Die entsprechende cDNA-Bank kann durch Isolierung von mRNA aus einer Gewebeprobe und anschließende Reverse-Transkription in an sich bekannter Weise erhalten werden. Aus der Nukleinsäuresequenz kann mit Hilfe des genetischen Codes die Aminosäuresequenz abgeleitet werden. Alternativ ist es hierzu auch möglich, homologe Sequenzen in EST (Expressed Sequence Tags)-Datenbanken zu suchen und zu kombinieren.

Die erfindungsgemäßen Nukleinsäuren eignen sich zur Expression des humanen und murinen KDR Rezeptors, sowie seiner splice-Varianten in pro- oder eukaryontischen Systemen. Darüber hinaus sind sie auch zur Expression des KDR Rezeptors in vivo im Sinne einer Gentherapie oder, insbesondere in Form von Fragmenten, auch in komplementärer Struktur als Antisense-Nukleotide zur Verringerung der Expression des KDR Rezeptors geeignet.

Die erfindungsgemäßen Nukleinsäuren können durch chemische Synthese oder durch Vervielfältigung in gentechnisch veränderten Organismen nach dem Fachmann an sich bekannten Verfahren hergestellt werden.

Gegenstand der Erfindung sind auch die durch die Expression der erfindungsgemäßen Nukleinsäuren erhältlichen KDR Rezeptor Proteine.

Die erfindungsgemäßen KDR Proteine lassen sich durch Expression in gentechnisch veränderten Organismen herstellen. Insbesondere sind eukaryontische Expressionssysteme geeignet. Entsprechende eukaryontische Expressionssysteme sind dem Fachmann bekannt wie beispielsweise pRc/CMV (Firma Stratagene). Die Aufreinigung aus gentechnisch veränderten Organismen bietet, insbesondere im Falle der Überexpression, ein leichten und direkten Zugang zu den erfindungsgemäßen KDR Rezeptoren, sowie zu deren Fragmenten, und erlaubt darüber hinaus die Isolierung in größeren Mengen.

Weiterhin beansprucht werden Varianten des KDR Rezeptors. Unter den Begriff "Varianten" fallen sowohl natürlich vorkommende allelische Variationen des KDR Rezeptors sowie durch rekombinante DNA-Technologie (insbesondere durch in vitro Mutagenese mit Hilfe von chemisch synthetisierten Oligonukleotiden) und anschließende Expression erzeugte Proteine, die hinsichtlich ihrer biologischen und/oder immunologischen Aktivität dem KDR Rezeptor oder dessen Fragmenten entsprechen. Dabei können sowohl Aminosäuren deletiert, eingefügt oder konservativ ausgetauscht werden. Konservativer Austausch bedeutet, dass eine Aminosäure durch eine Aminosäure ersetzt wird, die ähnliche physikalischchemische Eigenschaften aufweist.

So sind beispielsweise folgende Aminosäuren austauschbar: Serin für/gegen Alanin, Alanin für/gegen Glycin, Methionin für/gegen Serin, Lysin für/gegen Arginin, Lysin für/gegen Serin.

Insbesondere umfasst der Begriff Varianten auch N- und/oder C-terminale verkürzte Proteine sowie acetylierte, glykosylierte, amidierte und/oder phosphorylierte Derivate. Auch Verbindungen, bei denen der KDR Rezeptor oder seine Varianten mit weiteren Molekülen wie Farbstoffen, Radionukliden oder Affinitätskomponenten gekoppelt sind, stellen erfindungsgemäße Varianten dar.

Weitere erfindungsgemäße Varianten sind Konstrukte, bei denen das N-terminale Signalpeptid des KDR Rezeptors entfernt wurde, bzw. durch ein heterologes Signalpeptid ersetzt wird. Solche heterologen Signalpeptide sind Oligopeptide zwischen 1-50 Aminosäuren, bevorzugt zwischen 15-25 Aminosäuren. Sie sind in der Lage, die Expressionseigenschaften des KDR Rezeptors zu modulieren, oder das resultierende Protein an einen gewünschten Ort innerhalb oder außerhalb der exprimierenden Zelle zu leiten.

Weitere erfindungsgemäße Varianten sind Konstrukte, bei denen der C-terminus des KDR Rezeptors durch eine heterologe Oligomerisierungsdomäne ersetzt wurde. Bevorzugt handelt es sich um Konstrukte, bei denen der extrazelluläre N-terminale Anteil des KDR Rezeptor mit einem Peptid der Protein fusioniert wird, das eine Dimerisierung, Trimerisierung, oder höhere Oligomerisierung vermitteln kann. Die durch Expression entsprechender Nukleinsäuren erhaltenen artifiziellen KDR Varianten sind in der Lage, den Liganden des KDR Rezeptors zu binden. Die Oligomerisierung des löslichen Rezeptor-Konstruktes bewirkt dabei eine erhöhte Affinität zum Liganden.

Beansprucht werden auch Nukleinsäuren, die für den KDR Rezeptor kodieren bzw. komplementär zu diesen Nukleinsäuren sind. Bei den Nukleinsäuren kann es sich beispielsweise um DNA, RNA, PNA oder um nukleaseresistente Analoga handeln. Nukleaseresistente Analoga sind insbesondere solche Verbindungen, in denen die Phosphodiesterbindung durch hydrolysestabile Verbindungen modifiziert sind, beispielsweise Phosphothioate, Methylphosphonate o.ä.

Für Antisensenukleotide sind insbesondere kurze Fragmente der Nukleinsäuren geeignet. Diese sollten aus Gründen der Spezifität bevorzugt mehr als 6, noch mehr bevorzugt mehr als 8 und am meisten bevorzugt mehr als 12 Nukleotide aufweisen. Aus Gründen der Diffusion und der Kosten haben sie üblicherweise eine Länge von weniger als 30 Nukleotiden, bevorzugt 24 oder weniger und noch mehr bevorzugt 18 oder weniger Nukleotide.

Gegenstand der Erfindung sind auch Derivate von Nukleinsäuren, die für diagnostische oder therapeutische Zwecke mit anderen Molekülen gekoppelt sind, beispielsweise mit Fluoreszenzfarbstoffen, radioaktiven Markern oder Affinitätskomponenten, sowie Fragmente der erfindungsgemäßen Nukleinsäuren und der zu diesen Nukleinsäuren komplementären Nukleinsäuren sowie Varianten der Nukleinsäuren.

Fragmente bezeichnet dabei Nukleinsäuren, die am 5' oder 3' oder an beiden Seiten verkürzt sind. Unter dem Begriff "Varianten" wird verstanden, dass diese Nukleinsäuren unter stringenten Bedingungen mit der erfindungsgemäßen Nukleinsäure bzw. dazu komplementären Nukleinsäuren hybridisieren. Unter dem Begriff "stringente Bedingungen" wird verstanden, dass die Hybridisierung bei Bedingungen durchgeführt wird, bei der die Temperatur noch bis zu 10°C unter der Temperatur liegt (bei sonst identischen Bedingungen), bei der exakt komplementäre Nukleinsäuren gerade noch hybridisieren würden. Wenn beispielsweise eine exakt hybrisierende Nukleinsäure unter gegebenen Bedingungen bis zu einer Temperatur von ca. 55°C hybridisiert, dann sind stringente Bedingungen Temperaturen gleich oder höher 45°C. Bevorzugt ist der Temperaturbereich für stringente Bedingungen von 5°C, noch mehr bevorzugt von 3°C.

Desweiteren betrifft die Erfindung Antikörper, die gegen den erfindungsgemäßen KDR Rezeptor, dessen Varianten, oder die erfindungsgemäßen Nukleinsäuren gerichtet sind. Diese Substanzen eignen sich insbesondere zum Einsatz in der Diagnostik durch dem Fachmann an sich bekannten Immunoassays, zur histologischen Untersuchung sowie als Arzneimittel zur Behandlung von Zuständen, die mit einer Überexpression des KDR Rezeptors verbunden sind. Solche erfindungsgemäßen Antikörper können mit dem Fachmann an sich bekannten Verfahren durch Immunisierung mit KDR Rezeptor, erfindungsgemäßen Nukleinsäuren oder Peptid- und Nukleinsäurenfragmenten in Gegenwart von Hilfsreagenzien erhalten werden.

Weiterhin sind Gegenstand der Erfindung Zelllinien, die den erfindungsgemäßen KDR Rezeptor überexprimieren. Solche Zelllinien sind erhältlich durch Transfektion mit Vektoren, die die erfindungsgemäßen Nukleinsäuren, die für den KDR Rezeptor kodieren, enthalten. Im Falle von eukaryontischen Zelllinien kann die Transfektion beispielsweise durch Elektroporation erfolgen. Die Zelllinien sind dabei vorzugsweise stabil transfiziert.

Überexpression bedeutet in diesem Zusammenhang, dass diese Zelllinie eine erhöhte Menge des KDR Rezeptors aufweist als die Zelllinien, die nicht mit den erfindungsgemäßen Nukleinsäuren transfiziert wurden. Geeignete eukaryontische Zelllinien sind beispielsweise die Zelllinien U937, HEK 293 oder Jurkat.

Gegenstand der Erfindung ist weiterhin ein transgenes Säugetier, das eine Überexpression (gain of function) oder eine Gendefizienz bzw. einen Gendefekt (loss of function) für die den KDR Rezeptor, bzw. eine Variante des KDR Rezeptors, aufweist. Bevorzugt handelt es sich bei dem Säugetier um ein Nagetier, insbesondere eine Maus. Diese transgenen Säugetiere sind durch für den Fachmann an sich bekannte Verfahren erhältlich und eignen sich insbesondere zur Funktionsaufklärung des KDR Rezeptors. Für transgene Säugetiere werden definierte Genkonstrukte durch DNA-Mikroinjektion in den Vorkern (Pronukleus) einer befruchteten Eizelle im Einzellstadium injiziert, um die Expression des zusätzlichen Gens zu erreichen. Durch zielgerichtete Veränderung eines Gens im Genoms von ES-Zellen, die nachfolgend in Blastozysten injiziert werden, wird die Funktion eines Gens ausgeschaltet.

Bevorzugt handelt es sich bei den transgenen Tieren um Tiere, bei denen das Gen zeitlich und gewebsspezifisch von außen induzierbar ein- bzw. ausgeschaltet werden kann. Entsprechende transgene Säugetiere eignen sich insbesondere zur Aufklärung der mit dem erfindungsgemäßen KDR Rezeptor im Zusammenhang stehenden Stoffwechsel- und Signaltransduktionswegen, die wiederum diagnostische oder therapeutische Anwendungen eröffnen. Insbesondere eignen sich die transgenen Säugetiere zum Screening von pharmazeutischen Wirkstoffen.

Der erfindungsgemäße KDR Rezeptor, seine Varianten, die erfindungsgemäßen Nukleinsäuren, sowie die erfindungsgemäßen Antikörper können in Arzneimitteln und Diagnostikmitteln gegebenenfalls zusammen mit weiteren Hilfsstoffen enthalten sein. Diese Arznei- und Diagnostikmittel eigenen sich zur Diagnose und Behandlung von Erkrankungen, die auf einer Über- oder Unterexpression und/oder einer erhöhten oder verminderten Liganden-Bindung des KDR Rezeptors und/oder auf Störungen der Zellproliferation, Zelldifferenzierung und/oder Apoptose beruhen.

Insbesondere sind dies Erkrankungen, bei denen Entzündungsprozesse, Zellwachstumsstörungen und Immun-Reaktionen eine Rolle spielen. Dies können beispielsweise Krebserkrankungen, lokalisierte oder generalisierte Entzündungen, Immundefizienz oder Auto-Immunreaktionen sein.

Eine Reihe der möglichen Anwendungen des erfindungsgemäßen KDR Rezeptors ist in den folgenden Beispielen erläutert.

### Beispiel 1

### Diagnostische Anwendung

Eine Reihe von Krankheiten, insbesondere Leukämien, Tumor-Erkrankungen und entzündliche Erkrankungen, sind durch eine Veränderung der KDR Expression gekennzeichnet. Neben der Gesamtmenge des exprimierten KDR Rezeptors variieren auch die relativen Anteile der splice-Varianten. Die Messung des Expressionsgrades der verschiedenen KDR Varianten kann daher zur Diagnose solcher Krankheiten herangezogen werden.

Die Messung des KDR Expressionsgrades kann mit einer Reihe von unterschiedlichen, dem Fachmann bekannten Methoden erfolgen. Zum Beispiel kann die Hybridisierung mit einer KDR-spezifischen Sonde, die aus den hier offenbarten Nukleinsäuresequenzen abgeleitet wurde, in quantitativer Weise erfasst werden. Alternativ können quantitative Varianten der Polymerase Kettenreaktion mit KDR-spezifischen Primern durchgeführt werden. Die dafür benötigten Primer können ebenfalls aus den Sequenzen mit Seq. ID 2,4,6,8,10,12,14,16 abgeleitet werden, indem sie Fragmenten dieser Sequenzen entsprechen oder zu ihnen komplementär sind.

### Beispiel 2

### Screening nach Rezeptor Agonisten und Antagonisten

Die offenbarten Sequenzen des KDR Rezeptors können dazu verwendet werden, Moleküle zu identifizieren, die an den KDR Rezeptor binden. Solche Moleküle können entweder Agonisten sein, die analog zum natürlichen Liganden an den Rezeptor binden und eine physiologische Funktion des Rezeptor hervorrufen. Es kann sich aber auch um Antagonisten handeln, die den natürlichen Liganden vom Rezeptor verdrängen und so eine Funktion des Rezeptor inhibieren können.

Zur Suche nach Agonisten und Antagonisten wird, basierend auf den offenbarten Nukleinsäure-Sequenzen, ein Konstrukt erstellt, in dem die Liganden-Bindungsdomäne des KDR Rezeptors mit einer geeignete Reporter-Domäne fusioniert wird. Das Konstrukt kann dann in eukaryotischen Zellen, prokaryotischen Zellen, oder zellfreien Systemen exprimiert werden und mit Kandidaten-Substanzen in Kontakt gebracht werden. Die Konformationsänderung, die bei einer Bindung der getesteten Substanz an den Rezeptor auftritt, wird von der Reporter-Domäne in ein detektierbares Signal umgewandelt.

Zu diesem Zweck können verschiedenartige Reporterdomänen eingesetzt werden, einschliesslich solchen, deren Fluoreszenz-Eigenschaften sich bei einer Konformationsänderung in detektierbarer Weise verändern. Die Substanzen, die auf Bindung an den Rezeptor getestet werden können, schließen, neben niedermolekularen Verbindungen, auch natürlich vorkommende Proteine und Peptide ein, sowie künstlich erzeugte oder modifizierte Protein- und Peptid-Derivate.

Eine weitere Methode zur Suche nach Agonisten und Antagonisten beruht auf der Immobilisierung der Kandidatensubstanzen an einem festen oder Gelartigen Trägermaterial oder an der Oberfläche von Zellen oder Viren. Die immobilisierten Substanzen werden dann mit dem KDR Rezeptor, oder Teilen davon, in Kontakt gebracht. Die Bindung des Rezeptors kann z.B. durch Bindung eines detektierbaren Antikörpers quantifiziert werden.

### Beispiel 3

### Antikörper

Rezeptoren aus der TNF-Rezeptor Familie, zu der auch der KDR Rezeptor gehört, werden bei Ligandenbindung durch eine Konformationsänderung aktiviert und/oder deaktiviert. Eine analoge Konformationsänderung lässt sich auch durch die Bindung bestimmter Antikörper an den Rezeptor hervorrufen.

Die offenbarten Peptidsequenzen, bzw. Teile davon, können mit dem Fachmann bekannten Verfahren zur Gewinnung von spezifischen Antikörpern verwendet werden. Bevorzugt handelt es sich bei den Antikörpern um monoklonale Antikörper. Die gewonnenen Antikörper können dann in einem Testsystem, analog dem in Beispiel 2 beschriebenen, auf Bindung an den Rezeptor und Aktivierung/Deaktivierung des Rezeptors geprüft werden. Die Bindung der Antikörper an den Rezeptor kann auch mit bekannten Immunologischen Methoden, wie z.B. ELISA, Western Blot, und Oberflächen Plasmonen-Resonanz (SPR) überprüft werden.

Geeignete Antikörper können therapeutisch zur positiven und/oder negativen Beeinflussung des KDR Rezeptors verwendet werden.

### Beispiel 4

### Gentherapie

Krankheiten, die durch eine verminderte Expression des KDR Rezeptors gekennzeichnet sind, können durch eine künstlich erzeugte Produktion des KDR Rezeptors im Organismus therapeutisch beeinflusst werden. Zu diesem Zweck können die offenbarten Nukleinsäure-Sequenzen verwendet werden, indem sie mit Hilfe eines viralen oder nicht-viralen Systems zur Transfektion in den zu therapierenden Organismus eingebracht werden. Durch die Wahl eines geeigneten Gewebe-spezifischen Promotors kann die Expression des KDR Rezeptors gesteuert werden.

### Beispiel 5

### Therapie mit löslichen KDR Konstrukten

Krankheiten, die durch eine verstärkte Aktivität des KDR Rezeptors gekennzeichnet sind, können durch die spezifische Inhibition des KDR-Liganden therapiert werden. Die hier offenbarten Sequenzen ermöglichen einen solchen Therapie-Ansatz auch ohne Kenntnis der Natur des KDR-Liganden. Die in Seq. ID 3-8 und 11-16 gegebenen Protein- und Nukleotidsequenzen entsprechen löslichen Formen des KDR Rezeptors, die den entsprechenden Liganden binden können, ohne jedoch das resultierende Signal ins Innere der exprimierenden Zelle weiterzuleiten. Sie haben deshalb einen inhibitorischen Effekt auf die KDR-Signaltransduktion.

Zum Zwecke einer therapeutischen Reduktion des KDR Signals können z.B. Proteine gemäß Seq. ID 3 oder 5 oder 7 oder 11 oder 13 oder 15 dem Patienten oder dem Versuchstier verabreicht werden. Alternativ können Zelllinien oder andere Systeme konstruiert werden, die die Nukleinsäuren gemäß Seq. ID 4,6,8,12,14 oder 16 verwenden um die entsprechenden Proteine zu exprimieren.

Zur Steigerung der Wirkung der löslichen KDR Konstrukte ist es wünschenswert, eine Liganden-unabhängige Oligomerisierung der löslichen Rezeptoren zu erreichen. Zu diesem Zweck können, wie in Figur 1C gezeigt, heterologe Oligomerisierungsdomänen an den C-Terminus des Rezeptors fusioniert werden. Diese Domänen bewirken eine Oligomerisierung, bevorzugt eine Trimerisierung, des löslichen KDR Rezeptors. Es ist ebenfalls möglich, vor der C-terminalen Fusion der Oligomerisierungsdomäne den C-Terminus des KDR Rezeptor Konstruktes zu verkürzen. In einem solchen Konstrukt befindet sich die N-terminale Ligandenbindungs-Domäne des KDR Rezeptors, fusioniert mit einer Oligomerisierungsdomäne (Figure 1C).

### Beispiel 6

### Verwendung der KDR-eigenen Oligomerisierungsdomäne.

Wie in Beispiel 5 erläutert, bedürfen lösliche Formen von Rezeptoren der TNF-Rezeptor Familie einer Liganden-unabhängigen Oligomerisierung, bevorzugt einer Timerisierung.

Die splice-Varianten KDR-d3, KDR-d4 und KDR-d3d4 entsprechen an ihrem N-terminus dem kompletten KDR Rezeptor, weichen jedoch am C-terminus von der kanonischen KDR Sequenz ab. Die drei verkürzten Varianten enthalten jedoch an ihrem jeweiligen C-terminalen Ende Sequenzen, die eine Trimerisierung bewirken können (T1, T2, T3 in Figur 1A).

Auch für andere Mitglieder der TNF-Rezeptor Familie lassen sich lösliche inhibitorische Formen konstruieren, indem die jeweilige Liganden-Bindungsdomäne mit einer geeigneten Trimerisierungsdomäne fusioniert wird (analog zur in Beispiel 5 für KDR beschriebenen Vorgehensweise). Die C-termini T1, T2 und T3 der in Seq. ID 3,5,7,11,13,15 offenbarten Sequenzen eignen sich als solche Trimerisierungsdomänen. Sie können, wie in Figur 1B gezeigt, an heterologe Mitglieder der TNF-Rezeptor Familie fusioniert werden und so deren Trimerisierung fördern. Beispiele für Rezeptoren, bei denen diese Vorgehensweise möglich ist, sind TNF, Fas, die TRAIL-Rezeptoren und andere Mitglieder der TNF-Rezeptor Familie, wie sie in der Literatur dargestellt ist (Bodmer et al. 2002; Locksley et al. 2001).

### Beispiel 7

### mKDRectoFC-transgene Mäuse

Ziel war die Herstellung einer transgenen Maus, die die extrazelluläre Domäne des murinen KDR exprimiert, am C-terminalen Ende fusioniert mit einer humanen Fc-Domäne, die eine Multimerisierung des sezernierten Proteins erlaubt.

### 7a. Vektorkonstruktion und Herstellung der Transgenen

Die murine KDR cDNA, die für das Signalpeptid und die extrazelluläre Domäne des Proteins kodiert, wurde aus dem EST Klon BF452409 mittels PCR isoliert. Die eingesetzten Primer sind in der Tabelle 1 angegeben. Über die Primer wurde auf der 5' Seite eine HindIII Schnittstelle, und auf der 3' Seite des PCR-Produktes eine BamHI Schnittstelle für die weitere Klonierung eingefügt.

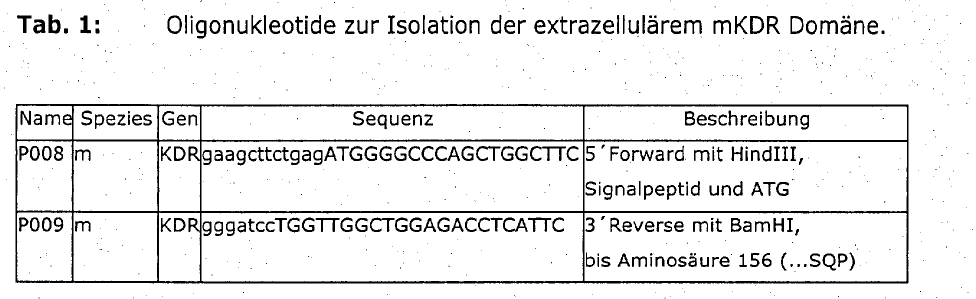

Das PCR-Produkt wurde in einen TA-Klonierungsvektor ligiert. Aus diesem wurde es über Restriktion mit den Enzymen HindIII und BamHI (Schnittstellen aus den Primern) ausgeschnitten und in den Vektor PS1121 ligiert. Dieser Vektor trägt eine FC-Domäne hinter der Multiple Cloning Site. Der entstandene PS1121 mKDRecto-PreScission-FC wurde mit HindIII/XhoI geschnitten, das isolierte Fragment mit Klenow behandelt und "Blunt end" in den über EcoRI linearisierten und mit Klenow behandelten Vektor PS750 kloniert (PS750 mKDRectoFCtg).

PS750 setzt sich zusammen aus dem humanen alpha1 anti-Trypsin Promotor (Nukleotide -6602 bis -5328) und dem Kaninchen beta-Globin Intron 2, Exon 3 und Poly A Signal (Nukleotid 421 - 1592) im pBluescript Vektor. Das mKDR ecto Fragment wird in das Exon 3 über die natürlich vorkommende EcoRI Schnittstelle ligiert.

Für die Eizellinjektion zur Herstellung der transgenen Tiere wurde die komplette Injektionskassette über einen Restriktionsverdau mit HindIII und XhoI ausgeschnitten.

Als Eizellspender dienten die Mausstämme C57/BL6 und FVB/n. Aus den Injektionen konnten drei Linien etabliert werden:
- Linie 1 im C57/BL6 Hintergrund
- Linien 29 im FVB/n Hintergrund
- Linie 44 im FVB/n Hintergrund

### 7b. Probenentnahme aus KDR transgenen Tieren und Wildtyp Geschwistern

Für die Analyse der Expression des KDR-Transgens wurden Mäuse aus der Linie 29 analysiert. Insgesamt wurden 4 Tiere aus einem Wurf präpariert: zwei transgene (Weibchen/Männchen) und zwei Wildtyp Mäuse (Weibchen/Männchen).

### a) Expressionsprofile Taqman

Insgesamt wurden 11 Gewebe auf die Expression von KDR hin in der Real Time PCR-Analyse (TaqMan) untersucht. Die Primer für die TaqMan Analyse wurden so ausgewählt, dass ein Fragment aus der Ecto-Domäne des Maus- KDR amplifiziert wird. Mit den Primern wird auf diese Weise sowohl das Wildtyp KDR als auch das transgene KDR detektiert. Je Gewebe wurden zwei PCRs durchgeführt.

In den Wildtyp Mäusen ist die Expression von KDR am höchsten in der Milz, gefolgt von Testis, Lunge und Dünndarm. In abnehmender Expressionsstärke folgen: Blut, Hirn, Leber, Niere, Magen, Dickdarm und Herz. Im Gegensatz dazu ist die Expression von KDR in der Niere und im Dünndarm der transgenen Tiere sehr stark erhöht (bis zu 1000fach in der Niere, 300fach im Dünndarm, s. Diagramm 1). Auch im Magen und im Dickdarm findet sich eine im Vergleich zum Wildtyp deutlich erhöhte Expression. Im Leber konnte eine leicht erhöhte Expression nachgewiesen werden (maximal 2fach), in allen anderen untersuchten Geweben entsprach die Expression im transgenen Tier der des Wildtyps.

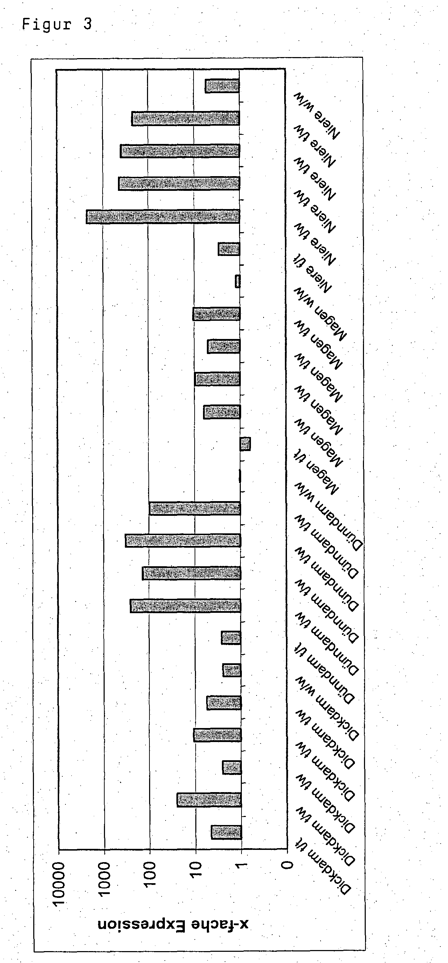

Figur 3 zeigt die Expression von KDR in transgenen (t) im Vergleich zu Wildtyp (W) Tieren (TaqMan-Analyse).

Neben dem Vergleich der Expression in der Transgenen im Vergleich zur Wildtyp Maus (t/w), wurde auch die KDR Expression in den zwei transgenen Mäusen (t/t) und in den zwei Wildtyp-Mäusen (w/w) verglichen.

### b) Western Blot Analyse

Zusätzlich wurde von verschiedenen Geweben eine Analyse der Expression auf Proteinebene durchgeführt. Zur Detektion des Proteins wurde ein von memorec hergestellter, monoklonaler Antikörper (Hybridom 1E2) eingesetzt, der zuvor auf stabilen Maus KDR- Zelllinien auf seine Spezifität getestet wurde.

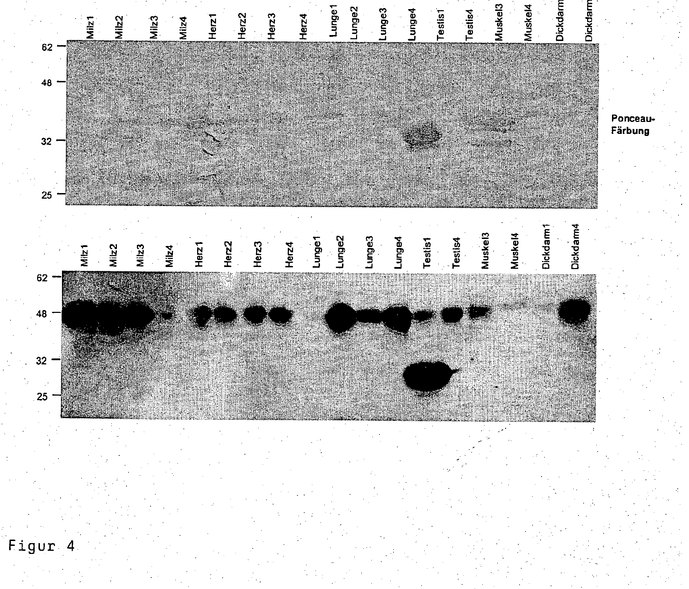

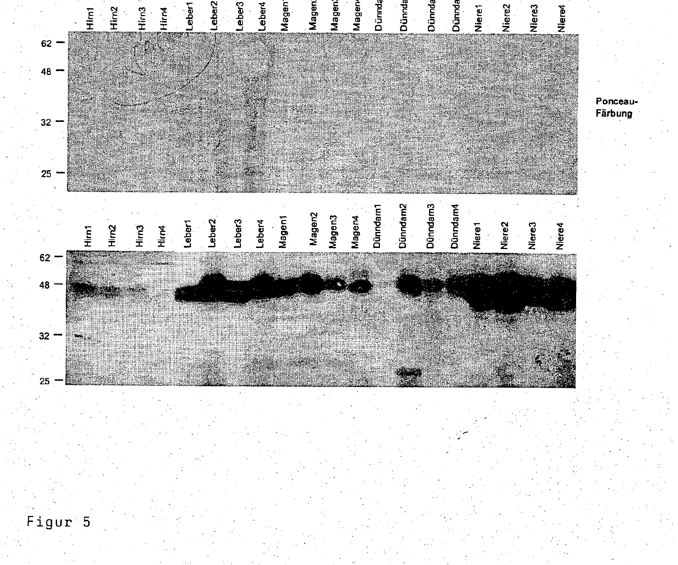

Während auf RNA-Ebene starke Expressionsunterschiede auftreten, weist der Western Blot auf eine von der Expression des Transgens fast unabhängige Expression in allen Geweben hin (Figuren 4 und 5). Im Gehirn ist die Expression von KDR deutlich geringer als in den anderen Geweben, aber auch in den transgenen Mäusen nicht erhöht.

Figur 4 zeigt eine Western Blot Analyse der KDR Expression in transgenen und Wildtyp-Mäusen (Blot 1).

Ponceau-Färbung zur Beladungskontrolle (oben) und Western Blot (unten). Hinter dem Namen des Gewebes ist die Nummer des jeweiligen Tieres vermerkt. 1 und 2 sind Wildtyp Tiere, 3 und 4 KDR transgene Tiere.

Im Dickdarm der transgenen Tiere (Dickdarm4, Figur 4) scheint die Menge an KDR Protein erhöht. Da auch bei den anderen Geweben starke individuelle Unterschiede auftreten, kann jedoch keine eindeutige Aussage getroffen werden.

Figur 5 zeigt die Western Blot Analyse der KDR Expression in transgenen und Wildtyp-Mäusen (Blot 2).

Ponceau-Färbung zur Beladungskontrolle (oben) und Western Blot (unten). Hinter dem Namen des Gewebes ist die Nummer des jeweiligen Tieres vermerkt. 1 und 2 sind Wildtyp Tiere, 3 und 4 KDR transgene Tiere.

### c) Immunfluoreszenz-Analyse

Mit dem gleichen Antikörper (1E2) wurde auch ein Immunfluoreszenz Analyse auf Gefrierschnitten durchgeführt. Dabei konnte in allen untersuchten Geweben (Gehirn, Herz, Lunge, Leber, Magen, Niere, Dünndarm) eine Zunahme der KDR Expression in den transgenen Tieren in Form einer Zunahme der Fluoreszenzintensität nachgewiesen werden.

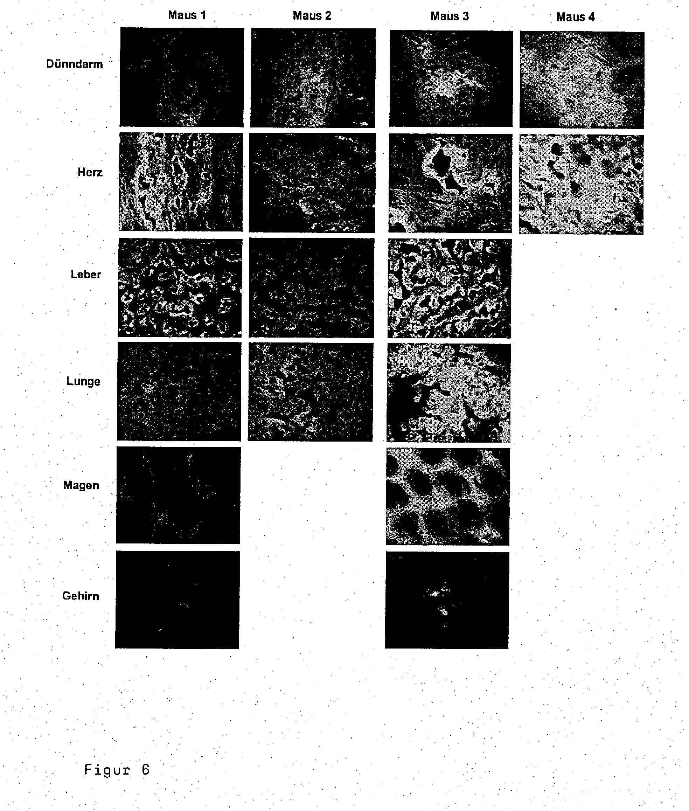

Figur 6 zeigt die Immunfluoreszenz- Analyse der KDR Expression.

Die Expression von KDR wurde in Gefrierschnitten von verschiedenen Geweben der Wildtyp- (Maus 1, Maus 2) und der KDR-transgenen Mäuse (Maus 3, Maus 4) untersucht.

### Literatur

Bodmer J., Schneider P., Tschopp J. (2002) The molecular architecture of the TNF superfamily. Trends Biochem. Sci. 27:19-26
Hofmann K.; Tschopp J. (1995) The death domain motif found in Fas (Apo-1) and TNF receptor is present in proteins involved in apoptosis and axonal guidance. FEBS Lett 371:321-323
Locksley R.M., Killeen N., Lenardo M.J. (2001) The TNF and TNF receptor superfamilies: integrating mammalian biology. Cell 104:487-501
Nagata S. (1997) Apoptosis by death factor. Cell 88:355-365
Schulze-Osthoff K., Ferrari D., Los M., Wesselborg S., Peter M.E. (1998) Apoptosis signaling by death receptors. Eur J Biochem 254:439-459

## Patentansprüche

1. Nukleinsäure kodierend für den humanen KDR Rezeptor gemäß Seq. ID2.

2. Nukleinsäure kodierend für eine lösliche Form des humanen KDR Rezeptors gemäß Seq. ID 4,6,8.

3. Nukleinsäure kodierend für den murinen KDR Rezeptor gemäß Seq. ID 10.

4. Nukleinsäure kodierend für eine lösliche Form des murinen KDR Rezeptors gemäß Seq. ID 12,14,16.

5. Nukleinsäure gemäß mindestens einem der Ansprüche 1 bis 4, **dadurch gekennzeichnet, dass** sie für einen Rezeptor der TNF-Rezeptor Familie kodiert.

6. Nukleinsäure gemäß mindestens einem der Ansprüche 1 bis 5 **dadurch gekennzeichnet, dass** es sich um DNA, RNA, PNA oder nukleaseresistente Analoga handelt.

7. Nukleinsäure gemäß mindestens einem der Ansprüche 1 bis 6 **dadurch gekennzeichnet, dass** es sich um mRNA, cDNA oder genomische DNA handelt.

8. Nukleinsäure **dadurch gekennzeichnet, dass** sie komplementär zur Nukleinsäure gemäß mindestens einem der Ansprüche 1 bis 7 ist.

9. Nukleinsäure gemäß mindestens einem der Ansprüche 1 bis 8, **dadurch gekennzeichnet, dass** es sich um Derivate, Fragmente oder Varianten dieser Nukleinsäuren handelt.

10. KDR Rezeptor erhältlich durch Expression der Nukleinsäure gemäß Anspruch 1 bis 8, insbesondere mit der Sequenz gemäß Seq. ID 1 oder Seq. ID 9.

11. Lösliche Formen des KDR Rezeptors gemäß Anspruch 10, insbesondere mit der Sequenz gemäß Seq. ID 3 oder 5 oder 7 oder 11 oder 13 oder 15.

12. Varianten und Fragmente des KDR Rezeptors gemäß mindestens einem der Ansprüche 10 bis 11.

13. Antikörper, **dadurch gekennzeichnet, dass** sie gegen den KDR Rezeptor gemäß mindestens einem der Ansprüche 10 bis 12 oder eine Nukleinsäure gemäß mindestens einem der Ansprüche 1 bis 9 gerichtet sind.

14. Antikörper gemäß Anspruch 13, **dadurch gekennzeichnet, dass** sie die Oligomerisierung des KDR Rezeptors gemäß mindestens einem der Ansprüche 10 bis 12 fördern können.

15. Antikörper gemäß Anspruch 13, **dadurch gekennzeichnet, dass** sie die Oligomerisierung des KDR Rezeptors gemäß mindestens einem der Ansprüche 10 bis 12 inhibieren können.

16. Zelllinie, **dadurch gekennzeichnet, dass** sie einen KDR Rezeptor gemäß mindestens einem der Ansprüche 10 bis 12 überexprimiert.

17. Transgenes Säugetier mit Überexpression (gain of function) oder Gendefizienz oder Gendefekt (loss of function) für einen KDR Rezeptor.

18. Transgenes Säugetier gemäß Anspruch 17 **dadurch gekennzeichnet, dass** es ein Nagetier ist.

19. Arzneimittel enthaltend einen KDR Rezeptor gemäß mindestens einem der Ansprüche 10 bis 12, eine Nukleinsäure gemäß mindestens einem der Ansprüche 1 bis 9 und/oder einen Antikörper gemäß mindestens einem der Ansprüche 13 bis 15, zusammen mit weiteren Hilfsstoffen.

20. Diagnostikmittel enthaltend einen KDR Rezeptor gemäß mindestens einem der Ansprüche 10 bis 12, eine Nukleinsäure gemäß mindestens einem der Ansprüche 1 bis 9 und/oder einen Antikörper gemäß mindestens einem der Ansprüche 13 bis 15, zusammen mit weiteren Hilfsstoffen.

21. Verwendung der Arzneimittel gemäß Anspruch 19 oder der Diagnostikmittel gemäß Anspruch 20 zur Diagnose und Behandlung von Erkrankungen, die auf einer Über- oder Unterexpression und/oder einer erhöhten oder verminderten Aktivität des KDR Rezeptors und/oder auf Störungen der Zellproliferation, Zelldifferenzierung und/oder Apoptose beruhen.

22. Verwendung gemäß Anspruch 21, **dadurch gekennzeichnet, dass** es sich bei den Erkrankungen um Entzündungsprozesse, Zellwachstumsstörungen, Krebs und/oder Immunstörungen wie Immundefizienz oder Autoimmunität handelt.

23. Verfahren zum Screening von Wirkstoffen **dadurch gekennzeichnet, dass** die Veränderung der Expression oder Aktivität des KDR Rezeptors in Zelllinien gemäß Anspruch 16 bei Zugabe von mindestens einer möglichen pharmazeutisch wirksamen Substanz gemessen wird.

24. Verwendung der Zelllinie gemäß Anspruch 16 zur Entwicklung und Prüfung von pharmazeutischen Leitstrukturen.

25. Verfahren zur Herstellung des KDR Rezeptors gemäß mindestens einem der Ansprüche 10 bis 12 durch chemische Peptidsynthese oder durch Expression in gentechnisch veränderten Organismen, insbesondere in eukaryontischen Expressionssystemen.


SEQUENCE LISTING

<110> MEMOREC Stoffel GmbH
<120> Der KDR Rezeptor und seine Anwendung
<130> none
<160> 16
<170> PatentIn version 3.1
<210> 1
<211> 355
<212> PRT
<213> Homo sapiens
<400> 1

Met Gly Pro Gly Arg Cys Leu Leu Thr Ala Leu Leu Leu Leu Ala Leu
1               5                   10                  15

Ala Pro Pro Pro Glu Ala Ser Gln Tyr Cys Gly Arg Leu Glu Tyr Trp
            20                  25                  30

Asn Pro Asp Asn Lys Cys Cys Ser Ser Cys Leu Gln Arg Phe Gly Pro
        35                  40                  45

Pro Pro Cys Pro Asp Tyr Glu Phe Arg Glu Asn Cys Gly Leu Asn Asp
    50                  55                  60

His Gly Asp Phe Val Thr Pro Pro Phe Arg Lys Cys Ser Ser Gly Gln
65                  70                  75                  80

Cys Asn Pro Asp Gly Ala Glu Leu Cys Ser Pro Cys Gly Gly Gly Ala
                85                  90                  95

Val Thr Pro Thr Pro Ala Ala Gly Gly Gly Arg Thr Pro Trp Arg Cys
            100                 105                 110

Arg Glu Arg Pro Val Pro Ala Lys Gly His Cys Pro Leu Thr Pro Gly
        115                 120                 125

Asn Pro Gly Ala Pro Ser Ser Gln Glu Arg Ser Ser Pro Ala Ser Ser
    130                 135                 140

Ile Ala Trp Arg Thr Pro Glu Pro Val Pro Gln Gln Ala Trp Pro Asn
145                 150                 155                 160

Phe Leu Pro Leu Val Val Leu Val Leu Leu Leu Thr Leu Ala Val Ile
                165                 170                 175

Ala Ile Leu Leu Phe Ile Leu Leu Trp His Leu Cys Trp Pro Lys Glu
            180                 185                 190

Lys Ala Asp Pro Tyr Pro Tyr Pro Gly Leu Val Cys Gly Val Pro Asn
        195                 200                 205

Thr His Thr Pro Ser Ser Ser His Leu Ser Ser Pro Gly Ala Leu Glu
    210                 215                 220

Thr Gly Asp Thr Trp Lys Glu Ala Ser Leu Leu Pro Leu Leu Ser Arg
225                 230                 235                 240

Glu Leu Ser Ser Leu Ala Ser Gln Pro Leu Ser Arg Leu Leu Asp Glu
                245                 250                 255

Leu Glu Val Leu Glu Glu Leu Ile Val Leu Leu Asp Pro Glu Pro Gly
            260                 265                 270

Pro Gly Gly Gly Met Ala His Gly Thr Thr Arg His Leu Ala Ala Arg
        275                 280                 285

Tyr Gly Leu Pro Ala Ala Trp Ser Thr Phe Ala Tyr Ser Leu Arg Pro
    290                 295                 300

Ser Arg Ser Pro Leu Arg Ala Leu Ile Glu Met Val Val Ala Arg Glu
305                 310                 315                 320

Pro Ser Ala Ser Leu Gly Gln Leu Gly Thr His Leu Ala Gln Leu Gly
                325                 330                 335

Arg Ala Asp Ala Leu Arg Val Leu Ser Lys Leu Gly Ser Ser Gly Val
            340                 345                 350

Cys Trp Ala
        355

<210> 2
<211> 1065
<212> DNA
<213> Homo sapiens
<400> 2

atggggcctg gacgatgcct cctgacggcc ttgttgcttc tggccctggc gccaccgccg 60

gaagcctccc agtactgcgg ccgccttgaa tactggaacc cagacaacaa gtgctgcagc 120

agctgcctgc aacgcttcgg gccgcccccc tgcccggact atgagttccg ggaaaactgc 180

ggactcaatg accacggcga tttcgtaacg cccccgttcc gaaagtgttc ttctgggcag 240

tgcaaccccg acggcgcgga gctatgtagc ccctgcggcg gcggagccgt gacccctact 300

cccgccgcgg gcgggggcag aaccccgtgg cgctgcagag agaggccggt ccctgccaag 360

gggcactgcc ccctcacacc tggaaaccca ggcgccccta gctcccagga gcgcagctca 420

ccagcaagtt ccattgcctg gaggacccct gagcctgtcc ctcagcaggc ctggccgaat 480

ttccttccgc tcgtggtgct ggtcctgctc ctgaccttgg cggtgatagc gatcctcctg 540

tttattctgc tctggcatct ctgctggccc aaggagaaag ccgaccccta tccctatcct 600

ggcttggtct gcggagtccc caacacccac accccttcct cctcgcatct gtcctcccca 660

ggcgccctgg agacagggga cacatggaag gaggcctcac tacttccact cctgagcagg 720

gaactgtcca gtctggcgtc acaacccctg tctcgcctcc tggatgagct ggaggtgctg 780

gaagagctga ttgtactgct ggaccctgag cctgggccag gtgggggtat ggcccatggc 840

actactcgac acctggccgc aagatatggg ctgcctgctg cctggtccac ctttgcctat 900

tcgctgaggc cgagtcgctc gccgctgcgg gctctgattg agatggtggt ggcaagggag 960

ccctctgcct ccctgggcca gcttggcaca cacctcgccc agctagggcg ggcagatgca 1020

ttgcgggtgc tgtccaagct tggctcatct ggggtttgct gggct 1065

<210> 3
<211> 71
<212> PRT
<213> Homo sapiens
<400> 3

Met Gly Pro Gly Arg Cys Leu Leu Thr Ala Leu Leu Leu Leu Ala Leu
1               5                   10                  15

Ala Pro Pro Pro Glu Ala Ser Gln Tyr Cys Gly Arg Leu Glu Tyr Trp
            20                  25                  30

Asn Pro Asp Asn Lys Cys Cys Ser Ser Cys Leu Gln Arg Phe Gly Pro
        35                  40                  45

Pro Pro Cys Pro Glu Ala Gly Pro Cys Gln Gly Ala Leu Pro Pro His
    50                  55                  60

Thr Trp Lys Pro Arg Arg Pro
65                  70

<210> 4
<211> 213
<212> DNA
<213> Homo sapiens
<400> 4
   atggggcctg gacgatgcct cctgacggcc ttgttgcttc tggccctggc gccaccgccg      60
   gaagcctccc agtactgcgg ccgccttgaa tactggaacc cagacaacaa gtgctgcagc     120
   agctgcctgc aacgcttcgg gccgcccccc tgcccggagg ccggtccctg ccaaggggca     180
   ctgccccctc acacctggaa acccaggcgc ccc                                   213

<210> 5
<211> 136
<212> PRT
<213> Homo sapiens
<400> 5

Met Gly Pro Gly Arg Cys Leu Leu Thr Ala Leu Leu Leu Leu Ala Leu
1               5                   10                  15

Ala Pro Pro Pro Glu Ala Ser Gln Tyr Cys Gly Arg Leu Glu Tyr Trp
            20                  25                  30

Asn Pro Asp Asn Lys Cys Cys Ser Ser Cys Leu Gln Arg Phe Gly Pro
        35                  40                  45

Pro Pro Cys Pro Asp Tyr Glu Phe Arg Glu Asn Cys Gly Leu Asn Asp
    50                  55                  60

His Gly Asp Phe Val Thr Pro Pro Phe Arg Lys Cys Ser Ser Gly Gln
65                  70                  75                  80

Cys Asn Pro Asp Gly Ala Glu Leu Cys Ser Pro Cys Gly Gly Gly Ala
                85                  90                  95

Val Thr Pro Thr Pro Ala Ala Gly Gly Gly Arg Thr Pro Trp Arg Cys
            100                 105                 110

Arg Glu Asn Cys Pro Val Trp Arg His Asn Pro Cys Leu Ala Ser Trp
        115                 120                 125

Met Ser Trp Arg Cys Trp Lys Ser
    130                 135

<210> 6
<211> 408
<212> DNA
<213> Homo sapiens
<400> 6

atggggcctg gacgatgcct cctgacggcc ttgttgcttc tggccctggc gccaccgccg 60

gaagcctccc agtactgcgg ccgccttgaa tactggaacc cagacaacaa gtgctgcagc 120

agctgcctgc aacgcttcgg gccgcccccc tgcccggact atgagttccg ggaaaactgc 180

ggactcaatg accacggcga tttcgtaacg cccccgttcc gaaagtgttc ttctgggcag 240

tgcaaccccg acggcgcgga gctatgtagc ccctgcggcg gcggagccgt gacccctact 300

cccgccgcgg gcgggggcag aaccccgtgg cgctgcagag agaactgtcc agtctggcgt 360

cacaacccct gtctcgcctc ctggatgagc tggaggtgct ggaagagc 408

<210> 7
<211> 167
<212> PRT
<213> Homo sapiens
<400> 7

Met Gly Pro Gly Arg Cys Leu Leu Thr Ala Leu Leu Leu Leu Ala Leu
1               5                   10                  15

Ala Pro Pro Pro Glu Ala Ser Gln Tyr Cys Gly Arg Leu Glu Tyr Trp
            20                  25                  30

Asn Pro Asp Asn Lys Cys Cys Ser Ser Cys Leu Gln Arg Phe Gly Pro
        35                  40                  45

Pro Pro Cys Pro Glu Leu Ser Ser Leu Ala Ser Gln Pro Leu Ser Arg
    50                  55                  60

Leu Leu Asp Glu Leu Glu Val Leu Glu Glu Leu Ile Val Leu Leu Asp
65                  70                  75                  80

Pro Glu Pro Gly Pro Gly Gly Gly Met Ala His Gly Thr Thr Arg His
                85                  90                  95

Leu Ala Ala Arg Tyr Gly Leu Pro Ala Ala Trp Ser Thr Phe Ala Tyr
            100                 105                 110

Ser Leu Arg Pro Ser Arg Ser Pro Leu Arg Ala Leu Ile Glu Met Val
        115                 120                 125

Val Ala Arg Glu Pro Ser Ala Ser Leu Gly Gln Leu Gly Thr His Leu
    130                 135                 140

Ala Gln Leu Gly Arg Ala Asp Ala Leu Arg Val Leu Ser Lys Leu Gly
145                 150                 155                 160

Ser Ser Gly Val Cys Trp Ala
                165

<210> 8
<211> 501
<212> DNA
<213> Homo sapiens
<400> 8

atggggcctg gacgatgcct cctgacggcc ttgttgcttc tggccctggc gccaccgccg 60

gaagcctccc agtactgcgg ccgccttgaa tactggaacc cagacaacaa gtgctgcagc 120

agctgcctgc aacgcttcgg gccgcccccc tgcccggaac tgtccagtct ggcgtcacaa 180

cccctgtctc gcctcctgga tgagctggag gtgctggaag agctgattgt actgctggac 240

cctgagcctg ggccaggtgg gggtatggcc catggcacta ctcgacacct ggccgcaaga 300

tatgggctgc ctgctgcctg gtccaccttt gcctattcgc tgaggccgag tcgctcgccg 360

ctgcgggctc tgattgagat ggtggtggca agggagccct ctgcctccct gggccagctt 420

ggcacacacc tcgcccagct agggcgggca gatgcattgc gggtgctgtc caagcttggc 480

tcatctgggg tttgctgggc t 501

<210> 9
<211> 344
<212> PRT
<213> Mus musculus
<400> 9

Met Gly Pro Ser Trp Leu Leu Trp Thr Val Ala Val Ala Val Leu Leu
1               5                   10                  15

Leu Thr Arg Ala Ala Ser Met Glu Ala Ser Ser Phe Cys Gly His Leu
            20                  25                  30

Glu Tyr Trp Asn Ser Asp Lys Arg Cys Cys Ser Arg Cys Leu Gln Arg
        35                  40                  45

Phe Gly Pro Pro Ala Cys Pro Asp His Glu Phe Thr Glu Asn Cys Gly
    50                  55                  60

Leu Asn Asp Phe Gly Asp Thr Val Ala His Pro Phe Lys Lys Cys Ser
65                  70                  75                  80

Pro Gly Tyr Cys Asn Pro Asn Gly Thr Glu Leu Cys Ser Gln Cys Ser
                85                  90                  95

Ser Gly Ala Ala Ala Ala Pro Ala His Val Glu Ser Pro Gly Arg Thr
            100                 105                 110

His Lys Gln Cys Arg Lys Pro Val Pro Pro Lys Asp Val Cys Pro Leu
        115                 120                 125

Lys Pro Glu Asp Ala Gly Ala Ser Ser Ser Pro Gly Arg Trp Ser Leu
    130                 135                 140
Gly Gln Thr Thr Lys Asn Glu Val Ser Ser Gln Pro Gly Phe Val Ser
145                 150                 155                 160

Ala Ser Val Leu Pro Leu Ala Val Leu Pro Leu Leu Leu Val Leu Leu
                165                 170                 175

Leu Ile Leu Ala Val Val Leu Leu Ser Leu Phe Lys Arg Lys Val Arg
            180                 185                 190

Ser Arg Pro Ser Ser Ser Ser Ala Phe Gly Asp Pro Ser Thr Ser Leu
        195                 200                 205

His Tyr Trp Pro Cys Pro Gly Thr Leu Glu Val Leu Glu Ser Arg Asn
    210                 215                 220

Arg Gly Lys Ala Asn Leu Leu Gln Leu Ser Ser Trp Glu Leu Gln Gly
225                 230                 235                 240

Leu Ala Ser Gln Pro Leu Ser Leu Leu Leu Asp Glu Leu Glu Val Leu
                245                 250                 255

Glu Glu Leu Ile Met Leu Leu Asp Pro Glu Pro Gly Pro Ser Gly Ser
            260                 265                 270

Thr Ala Tyr Gly Thr Thr Arg His Leu Ala Ala Arg Tyr Gly Leu Pro
        275                 280                 285

Ala Thr Trp Ser Thr Phe Ala Tyr Ser Leu Arg Pro Ser Arg Ser Pro
    290                 295                 300

Leu Arg Ala Leu Ile Glu Met Val Val Ala Arg Glu Pro Ser Ala Thr
305                 310                 315                 320

Leu Gly Gln Phe Gly Thr Tyr Leu Ala Gln Leu Gly Arg Thr Asp Ala
                325                 330                 335

Leu Gln Val Leu Ser Lys Leu Gly
            340

<210>  10
<211>  1032
<212>  DNA
<213>  Mus musculus
<400>  10

atggggccca gctggcttct ctggacagtg gcggtggcag tgctgctcct gacccgggct      60

gcgtcaatgg aagcctctag cttctgtggc caccttgagt actggaactc tgacaagagg     120

tgctgcagcc gctgcctgca acgctttggg cctcctgcat gtcctgatca cgagtttacg     180

gaaaactgcg ggctcaatga cttcggcgat actgtagcac atcctttcaa aaagtgttcc     240

cctgggtatt gcaaccccaa tggcacagag ctgtgtagcc agtgtagcag cggagccgcc     300

gcagccccag ctcacgtgga gagccctggt agaacccaca agcagtgtag aaagcccgtc     360

cctcccaagg atgtctgtcc tcttaaacct gaagacgcag gtgcctctag ctcacctggg     420

aggtggagcc ttgggcagac aaccaagaat gaggtctcca gccaaccagg ttttgtctca     480

gcctcagtgc tgcctctggc agtgttgcca ctgttgctgg tgctgcttct gatattggca     540

gtggtcttgc tctctttgtt caagagaaaa gtccgttccc gtcctagttc cagctcagct     600

tttggagatc ccagcacctc tctacattac tggccctgcc caggtaccct ggaggtattg 660

gaaagtagaa acagagggaa agctaatctg ctgcagctct caagctggga gcttcagggt 720

ctggcctctc agcccctctc cctcctgctg gatgagctgg aagttctgga ggagctgatt 780

atgctattgg accctgagcc tgggccgagc gggagcacgg cttatggtac cacacgacac 840

ctggctgcaa gatacgggct gcctgccacc tggtctacct ttgcctactc actgcggccc 900

agtcgctcac ccctgcgggc cctgattgag atggttgtgg caagggagcc ttctgctact 960

ctgggtcaat ttggcacata tttggctcag ctaggtcgca cagatgctct gcaggtgcta 1020

tctaaacttg gc 1032

<210> 11
<211> 65
<212> PRT
<213> Mus musculus
<400> 11

Met Gly Pro Ser Trp Leu Leu Trp Thr Val Ala Val Ala Val Leu Leu
1               5                   10                  15

Leu Thr Arg Ala Ala Ser Met Glu Ala Ser Ser Phe Cys Gly His Leu
            20                  25                  30

Glu Tyr Trp Asn Ser Asp Lys Arg Cys Cys Ser Arg Cys Leu Gln Arg
        35                  40                  45

Phe Gly Pro Pro Ala Cys Pro Ala Arg Pro Ser Gln Gly Cys Leu Ser
    50                  55                  60

Ser
65

<210> 12
<211> 195
<212> DNA
<213> Mus musculus
<400> 12

atggggccca gctggcttct ctggacagtg gcggtggcag tgctgctcct gacccgggct 60

gcgtcaatgg aagcctctag cttctgtggc caccttgagt actggaactc tgacaagagg 120

tgctgcagcc gctgcctgca acgctttggg cctcctgcat gtcctgcccg tccctcccaa 180

ggatgtctgt cctct 195

<210> 13
<211> 140
<212> PRT
<213> Mus musculus
<400> 13

Met Gly Pro Ser Trp Leu Leu Trp Thr Val Ala Val Ala Val Leu Leu
1               5                   10                  15

Leu Thr Arg Ala Ala Ser Met Glu Ala Ser Ser Phe Cys Gly His Leu
            20                  25                  30

Glu Tyr Trp Asn Ser Asp Lys Arg Cys Cys Ser Arg Cys Leu Gln Arg
        35                  40                  45

Phe Gly Pro Pro Ala Cys Pro Asp His Glu Phe Thr Glu Asn Cys Gly
    50                  55                  60

Leu Asn Asp Phe Gly Asp Thr Val Ala His Pro Phe Lys Lys Cys Ser
65                  70                  75                  80

Pro Gly Tyr Cys Asn Pro Asn Gly Thr Glu Leu Cys Ser Gln Cys Ser
                85                  90                  95

Ser Gly Ala Ala Ala Ala Pro Ala His Val Glu Ser Pro Gly Arg Thr
            100                 105                 110

His Lys Gln Cys Arg Lys Ser Phe Arg Val Trp Pro Leu Ser Pro Ser
        115                 120                 125

Pro Ser Cys Trp Met Ser Trp Lys Phe Trp Arg Ser
    130                 135                 140

<210>  14
<211>  420
<212>  DNA
<213>  Mus musculus
<400>  14

atggggccca gctggcttct ctggacagtg gcggtggcag tgctgctcct gacccgggct      60

gcgtcaatgg aagcctctag cttctgtggc caccttgagt actggaactc tgacaagagg     120

tgctgcagcc gctgcctgca acgctttggg cctcctgcat gtcctgatca cgagttcacg     180

gaaaactgcg ggctcaatga cttcggcgat actgtagcac atcctttcaa aaagtgttcc     240

cctgggtatt gcaaccccaa tggcacagag ctgtgtagcc agtgtagcag cggagccgcc     300

gcagccccag ctcacgtgga gagccctggt agaacccaca agcagtgtag aaagagcttc     360

agggtctggc ctctcagccc ctctccctcc tgctggatga gctggaagtt ctggaggagc     420

<210>  15
<211>  163
<212>  PRT
<213>  Mus musculus
<400>  15

Met Gly Pro Ser Trp Leu Leu Trp Thr Val Ala Val Ala Val Leu Leu
1               5                   10                  15

Leu Thr Arg Ala Ala Ser Met Glu Ala Ser Ser Phe Cys Gly His Leu
            20                  25                  30

Glu Tyr Trp Asn Ser Asp Lys Arg Cys Cys Ser Arg Cys Leu Gln Arg
        35                  40                  45

Phe Gly Pro Pro Ala Cys Pro Glu Leu Gln Gly Leu Ala Ser Gln Pro
    50                  55                  60

Leu Ser Leu Leu Leu Asp Glu Leu Glu Val Leu Glu Glu Leu Ile Met
65                  70                  75                  80

Leu Leu Asp Pro Glu Pro Gly Pro Ser Gly Ser Thr Ala Tyr Gly Thr
                85                  90                  95

Thr Arg His Leu Ala Ala Arg Tyr Gly Leu Pro Ala Thr Trp Ser Thr
            100                 105                 110

Phe Ala Tyr Ser Leu Arg Pro Ser Arg Ser Pro Leu Arg Ala Leu Ile
        115                 120                 125

Glu Met Val Val Ala Arg Glu Pro Ser Ala Thr Leu Gly Gln Phe Gly
    130                 135                 140

Thr Tyr Leu Ala Gln Leu Gly Arg Thr Asp Ala Leu Gln Val Leu Ser
145                 150                 155                 160

Lys Leu Gly


<210>  16
<211>  489
<212>  DNA
<213>  Mus musculus
<400>  16

atggggccca gctggcttct ctggacagtg gcggtggcag tgctgctcct gacccgggct      60

gcgtcaatgg aagcctctag cttctgtggc caccttgagt actggaactc tgacaagagg     120

tgctgcagcc gctgcctgca acgctttggg cctcctgcat gtcctgagct tcagggtctg     180

gcctctcagc cccctcccct cctgctggat gagctggaag ttctggagga gctgattatg     240

ctattggacc ctgagcctgg gccgagcggg agcacggctt atggtaccac acgacacctg     300

gctgcaagat acgggctgcc tgccacctgg tctaccttcg cctactcact tcggcccagt     360

cgctcacccc tgcgggccct gattgagatg gttgtggcaa gggagccttc tgctactctg     420

ggtcaattcg gcacatattt ggctcagcta ggtcgcacag atgctctgca ggtgctatct     480

aaacttggc                                                             489